Europäisches Patentamt

European Patent Office

Office européen des brevets

(11) Publication number: **0 182 160**
A1

# (12) EUROPEAN PATENT APPLICATION

(21) Application number: **85113832.1**

(22) Date of filing: **30.10.85**

(51) Int. Cl.⁴: **A 61 N 1/42, H 01 F 13/00**

(30) Priority: **09.11.84 IT 292284**

(43) Date of publication of application: **28.05.86**
Bulletin **86/22**

(84) Designated Contracting States: **AT BE CH DE FR GB LI LU NL SE**

(71) Applicant: **DABER S.R.L., Via Gaffurio, 15, I-24100 Bergamo (IT)**

(72) Inventor: **Brandimante, Bruno, Via Archimede, 19, I-00197 Roma (IT)**

(74) Representative: **Giambrocono, Alfonso, Dr. Ing. et al, Ing. A. Giambrocono & C. S.r.i. Via Rosolino Pilo 19/B, I-20129 Milano (IT)**

(54) **Equipment apt to emit a sequence of magnetic pulses of substantially semicircular shape.**

(57) The equipment according to the invention consists of a solenoid combined with a Graetz bridge that in its turn may be fed by a transformer.

The magnetic field emitted by the solenoid is of the pulsating type having substantially semicircular outline with peaks of 20 Gauss.

EP 0 182 160 A1

1

0182160

Description:

The present invention relates to an equipment apt to emit
a sequence of magnetic pulses of substantially semicircular
shape.

Said equipment is employable where it is necessary to dis
pose of a pulsating magnetic field in order to conclude
industrial researches and/or experimentations.

Moreover it was noted that the magnetic flux generated by
said apparatus favours the microcirculation of the blood
both in the hairbulb and in the skin, causing a slowing
down or a complete stop of the hair falling out.

The equipment according to the present invention is apt to
emit a sequence of magnetic pulses and consists of an oscil
lating circuit feeding a solenoid controlled so as to gene
rate a sequence of magnetic pulses of substantially semicircular shape.

This and other aims of the invention will be apparent to
those skilled in the art on perusing the following descrip
tion and claims; the invention is illustrated by way of a
non-limitative exemplification in the figures of the annexed
sheet of drawing, wherein:

Fig. 1 shows a block diagram of the equipment according to
the invention;
Fig. 2 is a structural arrangement;
Fig. 3 is a diagram showing the shape of the magnetic field,

2

generated by the equipment itself, with passing of time.

With reference to the above-mentioned figures, the equipment according to the invention, generally shown at 1, consists of a transformer 2 feeding the first couple of poles 3 and 4 of a Graetz bridge 5.

The remaining two poles 6 and 7, respectively positive and negative, are connected to the ends of a solenoid 8.

Between the bridge 5 and the solenoid 8 a timing device 9 is provided apt to cadence or rate the activity intervals of the equipment.

During the therapeutic type of use, the solenoid is being disposed around the head of the patient at the height of the forehead and of the nape of the neck so that the magne tic field generated by the solenoid itself may reactivate the microcirculation of the blood both in the hair bulb and in the skin, thus causing a slowing down or a complete stop of the hair falling out.

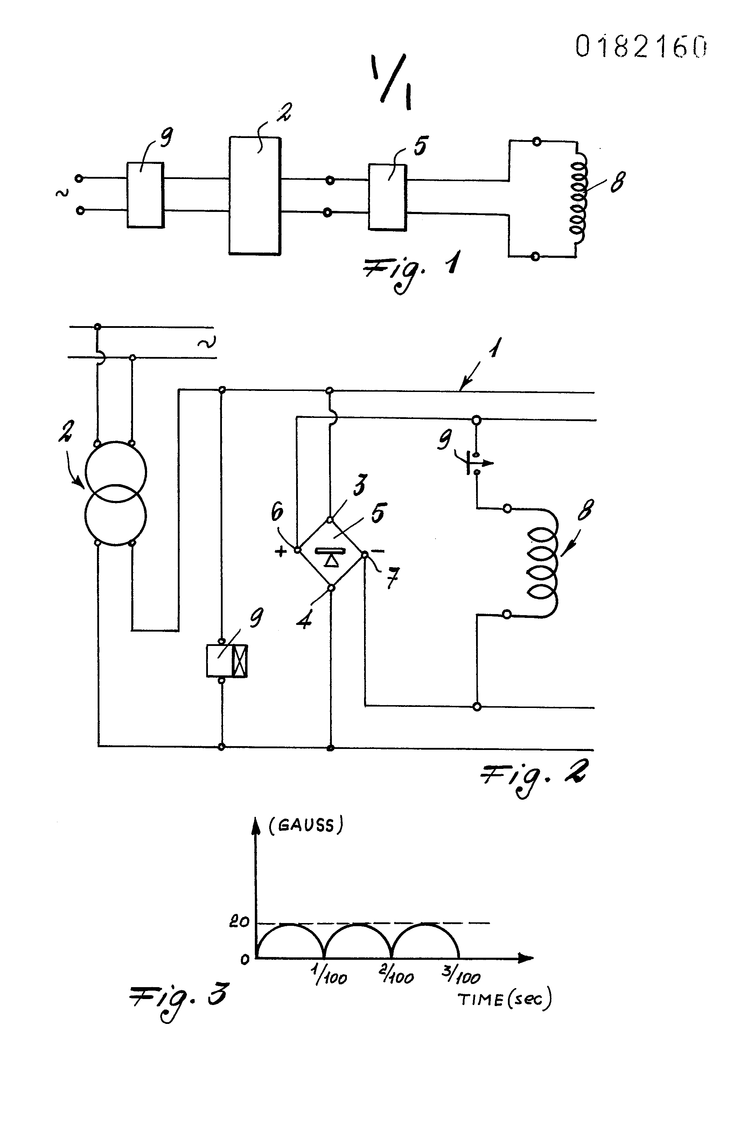

Said magnetic field has a maximum intensity of 20 Gauss (see figure 3) with a substantially semicircular outline and pulsation frequence of 100 Hz.

0182160

3

Claims:

1. An equipment apt to emit a sequence of magnetic pulses, characterized in that it consists of an oscillating circuit feeding a solenoid, said solenoid being controlled so as to generate a sequence of magnetic pulses of substantially semi circular shape.

2. An equipment according to the preceding claim, characte rized in that said oscillating circuit feeding the solenoid consists of a Graetz bridge that in its turn is fed by a transformer.

3. An equipment according to the preceding claims, characte rized in that a timing device apt to program the application treatment phases is provided.

4. An equipment according to the preceding claims, characte rized in that the signal emitted by the same shows a pulsation frequency of 100 Hz and a magnetic intensity from 0 to 20 Gauss.
All substantially as described, illustrated and claimed and for the above-specified purposes.

0182160

$^1/_1$

Fig. 1

Fig. 2

Fig. 3

(GAUSS)

20

0

$^1/_{100}$   $^2/_{100}$   $^3/_{100}$

TIME (sec)

European Patent
Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | FR-A-1 412 437 (ELMI) <br> * Page 2, the two last paragraphs; page 3, paragraphs 1-3 * | 1,2 | A 61 N 1/42 <br> H 01 F 13/00 |
| A | | 4 | |
| | --- | | |
| X | FR-A-2 046 145 (IIS ELECTROTEHNICA) <br> * Page 1, lines 14-38; page 2, lines 8-13; page 3, lines 20-28,35-40 * | 1-4 | |
| | --- | | |
| A | EP-A-0 048 451 (DROLET) <br> * Page 28, line 19 - page 29, line 18; page 36, lines 12-13; page 45, lines 17-21 * | 1,3,4 | |
| | ----- | | TECHNICAL FIELDS SEARCHED (Int. Cl.4) <br><br> A 61 N |

The present search report has been drawn up for all claims

| Place of search | Date of completion of the search | Examiner |
|---|---|---|
| THE HAGUE | 12-02-1986 | SIMON J.J.E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82